# EUROPEAN PATENT APPLICATION

(11) **EP 2 899 686 A1**
(43) Date of publication of application: **29.07.2015**
(21) Application number: 13839113.1
(22) Date of filing: 11.09.2013
(51) Int. Cl.: G06Q 50/22, A61B 5/00, A61B 5/16

(54) **MENTAL HEALTH CARE SUPPORT DEVICE, SYSTEM, METHOD, AND PROGRAM**

(30) Priority: 24.09.2012 JP 2012209523
(71) Applicant: NEC Solution Innovators, Ltd., Tokyo 136-8627 (JP)
(72) Inventor: YAMAMOTO, Kosuke, Tokyo 1368627 (JP); ONO, Yutaka, Tokyo 157-0066 (JP)
(74) Representative: Robson, Aidan John
(86) International application number: PCT/JP2013/005367
(87) International publication number: WO 2014/045546

(57) **Abstract**

A mental health care support device which carries out an accurate counseling is provided. The mental health care support device includes contradiction determination means for determining whether there is a contradiction between an automatic thought and a feeling, on the basis of an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category.

## Description

### Technical Field

The present invention relates to a mental health care support device, a mental health care support system, a mental health care support method and a mental health care support program which support a mental health care.

### Background Art

A cognition reconstruction method is exemplified as one of the cognitive behavior therapies each of which is a methodology for curing a habit of thinking in a bad direction to get a well-balanced way of thinking by focusing on a fact and a behavior. By writing down feelings which a subject person has at a time when the subject person encounters an event beating an own heart, thoughts which the subject person has at the time, a reason why the subject person think so, and the like in turn, the cognition reconstruction method makes it possible to review the way of thinking.

A patent literature 1 describes a mental health care system in which a state of a user's stress is acquired or measured, and counseling is carried out to the user in a dialogue mode by use of the measurement result.

### Citation List

### Patent Literature

PTL 1: Japanese Patent Application Laid-Open Publication No. 2005-334205

### Summary of Invention

### Technical Problem

It is difficult, however, for the counselor to acquire correct information on the feelings, the thoughts and the like which are required for the counseling, and to process the counseling accurately even if a counselor is skilled.

According to the cognition reconstruction method, thoughts of a counselee which occur at the time when the event beats the heart of the counselee are referred to as "automatic thoughts". In order to improve the feelings, it is important to identify the automatic thoughts which generate the feelings of the counselee. However, there is a problem that it is not always possible for the counselee to express the own automatic thoughts and the own feelings with correct words. Moreover, there is another problem that, even if the counselee can express the own automatic thoughts and the own feelings with the correct words, the counselor does not perceive that a contradiction between the automatic thoughts and the feelings of the counselee is generated, and consequently continues the counseling.

In the case that the contradiction between the automatic thoughts and the feelings of the counselee is generated, if the counselor has not correct perception on the contradiction, it is impossible to acquire an effect of improving the feelings. If the counselor is skilled, the counselor may perceive the contradiction as a feeling that something is wrong in some cases. However, there is a problem that the counselor who is not skilled, or a system which is automatized does not perceive the contradiction between the automatic thoughts and the feelings, and consequently continues the ineffective counseling.

For example, it is assumed that a counselee, who is a sales staff and fails in his business with a customer, insists to a counselor that the counselee "lost a customer's trust" and "feels angry". In this case, it is important that, regarding a linkage between "loss of customer's trust" and "feeling angry", the counselor has a feeling that something is wrong, and investigates furthermore why the counselor has the feeling that something is wrong.

However, in the case that the counselor continues the counseling without having the feeling that something is wrong, although it may be possible for the counselor to make the counselee perceive that "loss of customer's trust" is a mere groundless thought, it is impossible to assuage "anger" of the counselee since "loss of customer's trust" is not linked with the anger.

Meanwhile, it is supposed that the counselor can have correctly the feeling that something is wrong, and can investigate the feeling furthermore. In this case, it may be found that thought which causes the anger is, for example, that colleagues of company do not follow the counselee. Then, it is possible to set a problem for assuaging the feeling, for example, "How to construct a cooperative relation with the colleagues of company?".

Up to the present, detection of the contradiction between the automatic thoughts and the feelings depends on intuition of the counselor. In contrast, if there is a mechanism which can check automatically whether the contradiction between the automatic thoughts and the feelings is generated or not, it is conceivable that also a counselor who is not skilled enough, or an automatized system can carry out more suitable counseling.

Then, an object of the present invention is to provide a mental health care support device, a mental health care support system, a mental health care support method and a mental health care support program by which it is possible to carry out accurate counseling even in the case that a counselee cannot express own automatic thoughts and own feelings with correct words or in the case that a contradiction between the thoughts and the feelings of the counselee is generated.

### Solution to Problem

A mental health care support device according to an exemplary aspect of the present invention is characterized by including: contradiction determination means for determining whether there is a contradiction between an automatic thought and a feeling, on the basis of an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category.

A mental health care support device according to an exemplary aspect of the present invention is characterized by including, when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, outputting whether there is a contradiction between the automatic thought and the feeling.

A mental health care support device according to an exemplary aspect of the present invention is characterized by including, when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, rechecking the automatic thought or the feeling, or outputting an inquiry message for investigating the automatic thought or the feeling furthermore.

A mental health care support system according to an exemplary aspect of the present invention is characterized by including a server connected with a client terminal, which is operated by a counselee or a counselor, through a network; and
the server includes automatic thought information input means for being inputted an automatic thought expression expressing the automatic thought of the counselee or an automatic thought category in which the automatic thought of the counselee is classified into prescribed category from the client terminal through the network, feeling information input means for being inputted the feeling expression expressing the feeling of the counselee when the automatic thought occurs or a feeling category in which the feeling of the counselee is classified into prescribed category, contradiction determination means for determining whether there is a contradiction between an automatic thought and a feeling, on the basis of the automatic thought expression or the automatic thought category received by the automatic thought information input means and the feeling expression or the feeling category received by the feeling information input means, and determination result output means for outputting a determination result issued by the contradiction determination means to the client terminal through the network.

A mental health care support system according to an exemplary aspect of the present invention is characterized by including a server connected with a client terminal, which is operated by a counselee or a counselor, through a network; and
the server includes contradiction determination means for determining, when receiving an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category, whether there is a contradiction between an automatic thought and a feeling, on the basis of the received automatic thought expression or the automatic thought category, and the feeling expression or the feeling category.

A mental health care support method according to an exemplary aspect of the present invention is characterized by including, when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, determining whether there is a contradiction between the automatic thought and the feeling on the basis of the inputted automatic thought expression or the automatic thought category, and the feeling expression or the feeling category.

A mental health care support program according to an exemplary aspect of the present invention is characterized by causing a computer to execute: a process for, when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, determining whether there is a contradiction between the automatic thought and the feeling on the basis of the inputted automatic thought expression or the automatic thought category, and the feeling expression or the feeling category.

### Advantageous Effects of Invention

According to the present invention, even in the case that the counselee cannot express the own automatic thoughts and the own feelings with the correct words, or even in the case that there is the contradiction between the thoughts and the feelings of the counselee, it is possible to carry out the accurate counseling.

### Brief Description of Drawings

Fig. 1 is an explanation diagram explaining correspondences between thoughts and feelings.
Fig. 2 is a block diagram exemplifying a configuration of a mental health care support device of a first exemplary embodiment.
Fig. 3 is a flowchart exemplifying an operation of the mental health care support device of the first exemplary embodiment.
Fig. 4 is a block diagram exemplifying a configuration of a mental health care support device of a second exemplary embodiment.
Fig. 5 is an explanation diagram exemplifying an input interface of the second exemplary embodiment.
Fig. 6 is an explanation diagram showing an example of registering a correct solution set of an automatic thought expression and a feeling category.
Fig. 7 is an explanation diagram showing an example of registering a correspondence table which indicates a correspondence between an example of feeling expression, and the feeling category.
Fig. 8 is an explanation diagram exemplifying characteristics of the automatic thought expression per each category.
Fig. 9 is a flowchart exemplifying an operation of the mental health care support device of the second exemplary embodiment.
Fig. 10 is an explanation diagram exemplifying an output which indicates a determination result issued by a determination result output unit 4.
Fig. 11 is a block diagram exemplifying a configuration of a mental health care support device of a third exemplary embodiment.
Fig. 12 is a flowchart exemplifying an operation of the mental health care support device of the third exemplary embodiment.
Fig. 13 is a flowchart exemplifying an operation which is carried out in the case that the present invention is applied to a system which carries out self-counseling.
Fig. 14 is an image diagram of a screen exemplifying contents of a service according to the present invention.
Fig. 15 is an image diagram of a screen exemplifying contents of a service according to the present invention.
Fig. 16 is an image diagram of a screen exemplifying contents of a service according to the present invention.
Fig. 17 is an image diagram of a screen exemplifying contents of a service according to the present invention.
Fig. 18 is a block diagram exemplifying a configuration of a mental health care support system of the present invention.
Fig. 19 is a block diagram showing an outline of the present invention.
Fig. 20 is a block diagram showing an outline of the present invention.

### Description of Embodiments

### Exemplary embodiment 1

Hereinafter, an exemplary embodiment of the present invention will be explained with reference to a drawing.

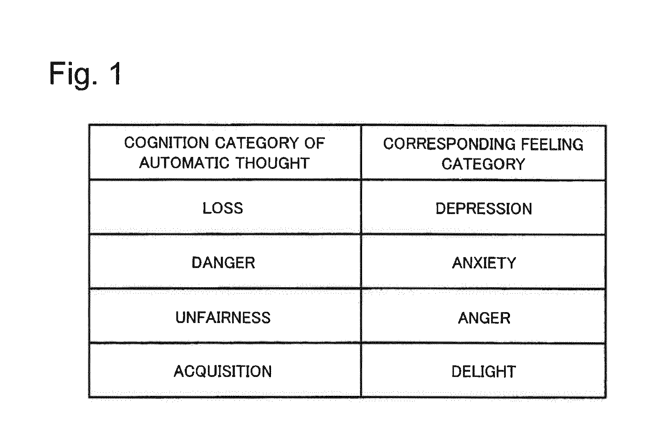

Firstly, a relation between automatic thought and feeling will be explained in the following. It is generally said that the automatic thought is related to four types of cognition, that is, "loss", "danger", "unfairness" and "acquisition". It is generally said that the negative automatic thought out of the four types of cognition is related to three types of cognition of "loss", "danger" and "unfairness", and the three types of cognition bring about feelings of "depression", "anxiety" and "anger" respectively. Here, a feeling corresponding to "acquisition" is "delight". It is generally said that, when the automatic thoughts and the feelings are classified into categories respectively as mentioned above, the category of the automatic thought and the category of the feeling have one to one correspondence. Fig. 1 is an explanation diagram explaining correspondences between the automatic thoughts and the feelings. Hereinafter, categories into which the automatic thoughts are classified from a cognition point of view as shown in Fig. 1 are called "automatic thought categories", and categories of feelings corresponding to the automatic thought categories are called "feeling categories". While only the negative automatic thought is considered to be an object of the automatic thought category according to the present invention, it is also possible to consider the positive automatic thought to be the object of the automatic thought category.

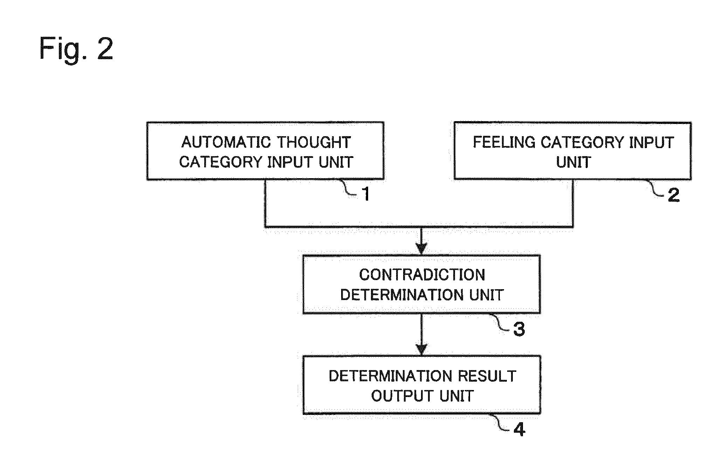

Next, a configuration of the mental health care support device of the exemplary embodiment will be explained in the following. Fig. 2 is a block diagram exemplifying the configuration of the mental health care support device of the exemplary embodiment. The mental health care support device shown in Fig. 2 includes an automatic thought category input unit 1, a feeling category input unit 2, a contradiction determination unit 3 and a determination result output unit 4.

The automatic thought category input unit 1 is an information input means which is inputted of an automatic thought category corresponding to a cognition category of an automatic thought related to a thought which occurs to a counselee when an event which beats a heart of the counselee happens. For example, by making a user select an automatic thought category to which an automatic thought occurring to the user is corresponding, through an interface which can input any one selected by the user out of three or four kinds of the automatic thought categories, the automatic thought category input unit 1 may receive the input of the automatic thought category.

The feeling category input unit 2 is an information input means which is inputted a feeling category corresponding to a category of a feeling which the counselee has when the automatic thought occurs. By making a user select a feeling category to which a feeling occurring to the user is corresponding, through an interface which can input any one selected by the user out of three or four kinds of the feeling categories, the feeling category input unit 2 may receive the input of the feeling category.

On the basis of the automatic thought categories which are inputted by the automatic thought category input unit 1, and the feeling categories which are inputted by the feeling category input unit 2, the contradiction determination unit 3 determines whether there is a contradiction between the automatic thoughts and the feelings or not. The contradiction determination unit 3 firstly acquires the feeling category which can be thought easily from the automatic thought category which the automatic thought category input unit 1 inputs. Then, the contradiction determination unit 3 determines whether there is a contradiction by determining whether the feeling category which is acquired from the automatic thought category, and the feeling category of the feeling which the user inputs are coincident. For example, in the case that the feeling category which is acquired from the automatic thought category, and the feeling category of the feeling which the user inputs are coincident to each other, the contradiction determination unit 3 determines that there is no contradiction. On the other hand, in the case that both of the feeling categories mentioned above are not coincident to each other, the contradiction determination unit 3 determines that there is a contradiction.

In order to acquire the feeling category which can be thought easily from the automatic thought category, the mental health care support device may stores beforehand a correspondence between the automatic thought category and the feeling category, the feeling category being able to be thought easily from the automatic thought category, as an "automatic thought category and feeling category" correspondence table. In this case, by reading the feeling category which is associated with the automatic thought category inputted by the user from the stored "automatic thought category and feeling category" correspondence table, the contradiction determination unit 3 can acquire the feeling category which is corresponding to the automatic thought.

The determination result output unit 4 outputs a determination result which is issued by the contradiction determination unit 3. In the case that the determination result indicates that there is no coincidence, the determination result output unit 4 may output additionally the feeling category, which is acquired from the automatic thought category and which is used in the contradiction determination process, in order to inform what kind of contradiction is generated.

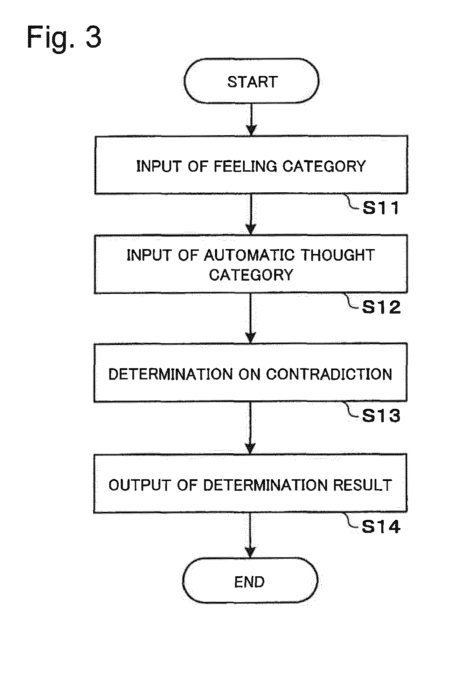

Fig. 3 is a flowchart exemplifying an operation of the mental health care support device of the exemplary embodiment. According to the example shown in Fig. 3, firstly, the feeling category input unit 2 receives inputs of the feeling category (Step S11). Next, the automatic thought category input unit 1 receives inputs of the automatic thought category (Step S12). When the feeling category and the automatic thought category are inputted, the contradiction determination unit 3 determines whether there is a contradiction between the automatic thought and the feeling of the counselee on the basis of the feeling category and the automatic thought category which are inputted (Step S13). Then, the determination result output unit 4 outputs the determination result which is issued by the contradiction determination unit 3(Step S14).

Here, the exemplary embodiment is not limited to the example shown in Fig. 3. For example, the automatic thought category may be inputted earlier than the feeling category is inputted, or the automatic thought category and the feeling category may be inputted at the same time.

As mentioned above, according to the present invention, since it is determined whether there is a contradiction between the automatic thoughts and the feelings or not, and the determination result is outputted, it is possible to make the counselor and the counselee perceive whether there is a contradiction between the automatic thoughts and the feelings or not, and consequently it is possible to support the accurate counseling.

### Exemplary embodiment 2

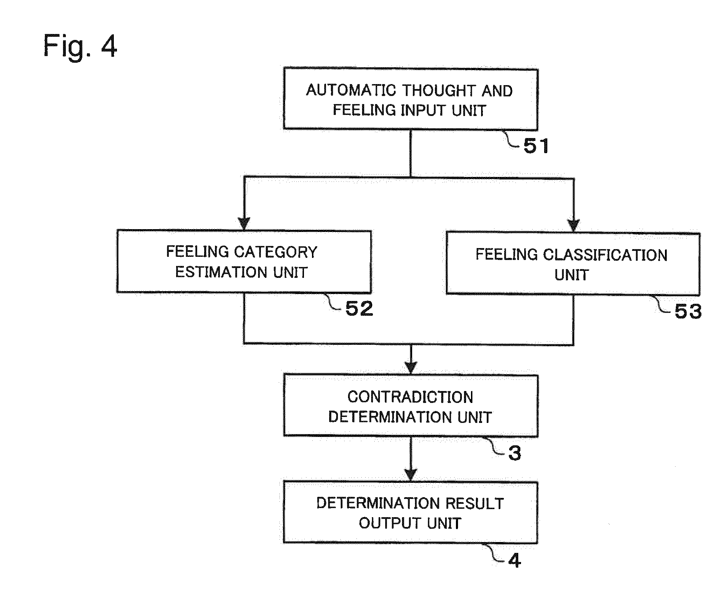

Next, a second exemplary embodiment of the present invention will be explained. Fig. 4 is a block diagram exemplifying a configuration of a mental health care support device of the second exemplary embodiment. The mental health care support device shown in Fig. 4 includes an automatic thought and feeling input unit 51, a feeling category estimation unit 52, a feeling classification unit 53, the contradiction determination unit 3 and the determination result output unit 4. Here, in comparison with the configuration of the first exemplary embodiment, the configuration of the exemplary embodiment is different in a point that the mental health care support device includes the automatic thought and feeling input unit 51, the feeling category estimation unit 52 and the feeling classification unit 53 in place of the automatic thought category input unit 1 and the feeling category input unit 2.

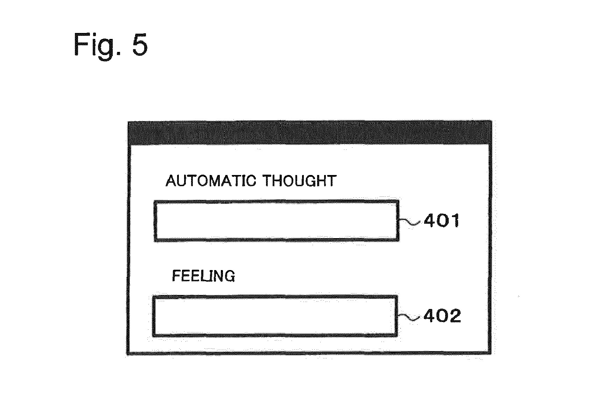

The automatic thought and feeling input unit 51 is an information input means for inputting information such as a text which expresses an automatic thought which occurs to a counselee when an event beats a heart of the counselee, and a feeling which the counselee has at the time. A user may make the automatic thought and feeling input unit 51 input the automatic thought and the feeling, which are exemplified in Fig. 5, through an interface through which the user can input automatic thoughts and feelings. Here, in the case that the counselee is a user, when inquiring of the counselee his thought and feeling directly, it is difficult that the user replies with no help of a counselor. Therefore, the automatic thought and feeling input unit 51 may inquire of the user the thought and the feeling simply in a way like "what did you think about at that time ?" or "what kind of feelings did you have at that time ?".

Fig 5 is an explanation diagram exemplifying an input interface of the exemplary embodiment. Fig. 5 shows a screen including an input column 401 and an input column 402 for writing the thought and the feeling respectively.

The feeling category estimation unit 52 extracts an expression (hereinafter, referred to as "automatic thought expression") expressing the automatic thought from the information which is received by the automatic and feeling input unit 51, and estimates a feeling category corresponding to the automatic thought of the counselee on the basis of the extracted automatic thought expression.

The feeling classification unit 53 extracts a feeling expression from the information which is received by the automatic and feeling input unit 51, and classifies the feeling, which the counselee had at that time, into the feeling category.

The contradiction determination unit 3 compares the feeling category which is estimated by the feeling category estimation unit 52, and the feeling category into which the feeling is classified by the feeling classification unit 53, and determines whether there is a contradiction between the automatic thought and the feeling of the counselee or not.

Here, according to the example mentioned above, the feeling category estimation unit 52 carries out up to the process of extracting the automatic thought expression and estimating the corresponding feeling category. Meanwhile, the feeling category estimation unit 52 may carry out up to the process of extracting the automatic thought expression and estimating the automatic thought category related to the extracted automatic thought expression. Afterward, similarly to the first exemplary embodiment, the contradiction determination unit 3 may estimate the feeling category which is corresponding to the estimated automatic thought category.

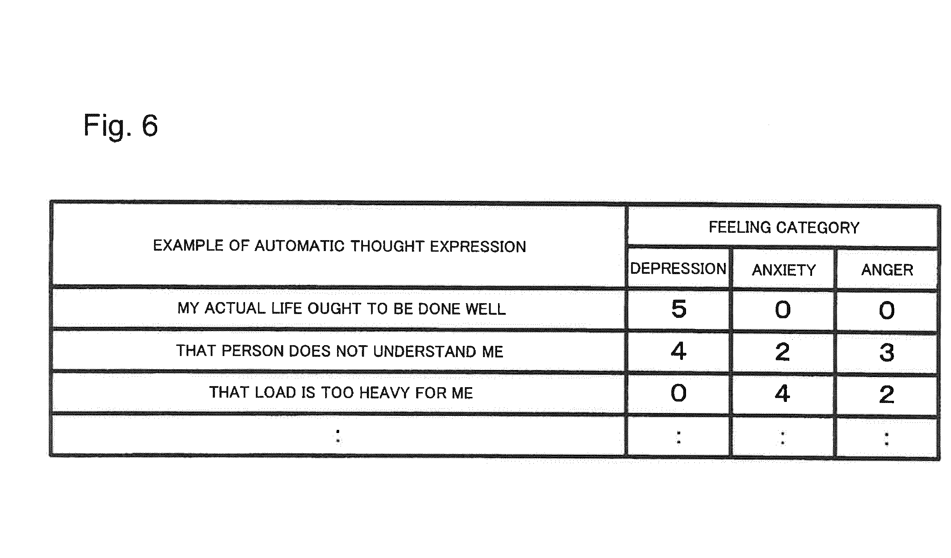

The feeling category estimation unit 52 may use, for example, the document classification art for estimating the feeling category or the automatic thought category. For example, as shown in Fig. 6, the feeling category estimation unit 52 may prepare a correct solution set, which indicates a degree of relation of the feeling category to the automatic thought expression, and may construct a statistical model regarding usage frequency of a word or sentence structure which is included in the automatic thought expression, and a value of the feeling category. By carrying out the above, the feeling category estimation unit 52 can estimate which feeling category each automatic thought is corresponding to. Moreover, the feeling category estimation unit 52 can estimate an automatic thought category from each automatic thought expression by associating the automatic thought expression with not the feeling category but the automatic thought category in the correct solution set.

Fig. 6 is an explanation diagram showing an example of registering the correct solution set of the automatic thought expression and the feeling category. According to the example shown in Fig. 6, a feeling category which is corresponding to an example of automatic thought expression is registered together with a probability value of each feeling category. In Fig. 6, a set of the probability values, which will be exemplified in the following, is stored. That is, a probability value of a probability that the feeling category, which is corresponding to an expression that "My actual life ought to be done better.", is a "depression" category is 5, and a probability value of a probability that the feeling category corresponding to the above-mentioned expression is an "anxiety" category is 0, and a probability value of a probability that the feeling category corresponding to the above-mentioned expression is an "anger" category is 0. Moreover, a probability value of a probability that the feeling category, which is corresponding to, for example, an expression that "That person does not understand me.", is the "depression" category is 4, and a probability value of a probability that the feeling category corresponding to the above-mentioned expression is the "anxiety" category is 2, and a probability value of a probability that the feeling category corresponding to the above-mentioned expression is the "anger" category is 3. Moreover, a probability value of a probability that the feeling category, which is corresponding to, for example, an expression that "That load is too heavy for me.", is the "depression" category is 0, and a probability value of a probability that the feeling category corresponding to the above-mentioned expression is the "anxiety" category is 4, and a probability value of a probability that the feeling category corresponding to the above-mentioned expression is the "anger" category is 2. Note that a maximum value of the probability value is "5" and minimum value of the probability value is "0" of each feeling category in Fig. 6.

Moreover, for classification which is carried out by the feeling classification unit 53, a correspondence table for converting an expression (hereinafter, referred to as "feeling expression"), which expresses the feeling and which is stored in advance, into the feeling category may be used.

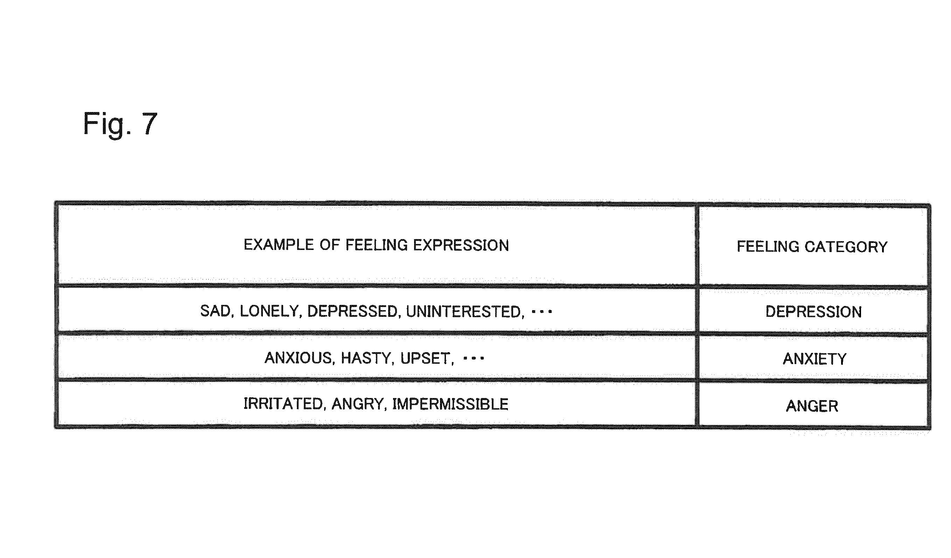

Fig. 7 is an explanation diagram showing an example of registering the correspondence table indicating a correspondence between an example of the feeling expression, and the feeling category. According to the example shown in Fig. 7, the feeling category which is corresponding to the example of the feeling expression is registered. In Fig. 7, for example, a "depression" category is registered correspondingly to expressions of, for example, "sad", "lonely", "depressed" and "uninterested". Moreover, for example, an "anxiety" category is registered correspondently to expressions of "anxious", "hasty" and "upset". Moreover, for example, an "anger" category is registered correspondently to expressions of "irritated", "angry" and "impermissible".

Moreover, Fig. 5 shows the example of receiving the automatic thought and the feeling separately. As another example, after receiving an input which describing freely an event beating a heart, the feeling category estimation unit 52 may regard a phrase, which is included in the input and which includes an expression such as "felt that..." or "thought that...", as the automatic thought, and may extract the phrase as the automatic thought. Moreover, the feeling classification unit 53 may extract an expression, which is regarded as "feeling", from the input by use of a dictionary or the like.

Moreover, the feeling category estimation unit 52 may use a classifier (model) such as a probability model or the like. Specifically, the feeling category estimation unit 52 can use a document classifier such as a generation model, an identification model or the like. As far as the model can identify which feeling category or which automatic though category the automatic thought is corresponding to, the classifier has no particular limitation. A classifier which can be used with ease according to a usage scene may be selected.

As an example of the generation model, Naive Bayes (Naive Bayes classifier) is exemplified. The feeling category estimation unit 52 may find out statistically in which feeling category a word is apt to be included on the basis of a stored pair of "automatic thought expression and feeling category" by use of Naive Bayes. By virtue of the above, it is possible to find out statistically to which feeling category an unknown automatic thought expression is apt to be corresponding. The generation model has an advantage that it is unnecessary to carry out re-learning to the category which has been stored, even if high speed learning is carried out and kinds of categories to be used become increasing.

Moreover, as an example of the identification model, SVM (Support Vector Machine) is exemplified. The feeling category estimation unit 52 may estimate a hyper-plane, which identifies the feeling category, in space of original characters of the automatic thought expression, for example, on the basis of a stored pair of "automatic thought expression and feeling category" by use of SVM. By mapping also an unknown automatic thought expression on the space, the feeling category estimation unit 52 can estimate to which feeling category the unknown automatic thought expression is corresponding. Since it is necessary to find a boundary which separates the categories in the case of a method which uses the identification model, when kinds of the categories become increasing, it is necessary to carry out re-learning. On the other hand, it is known that the identification model is generally more accurate than the generation model.

Hereinafter, the original characters, which are used when the feeling category estimation unit 52 carries out classification, will be explained in detail. Each cognition category which is the automatic thought category has a characteristic tendency regarding a way of thinking. For example, as cognition which is classified into a "loss" category, like an expression that "I have no bright future", there are many ways of thinking such that "I am spoiled", "Another person says that I am spoiled" or "My future is broken". Moreover, as cognition which is classified into a "crisis" category, like an expression that "A depressive surely cannot be restored to the former position", there are many ways of thinking such that "Danger is drawing near", "I am powerless" or "I have no support of another person". Moreover, as cognition which is classified into an "unfairness" category, like an expression that "The company robs me of my freedom", there are many ways of thinking such that "I think that I have no defect, but another person says that I am spoiled". The feeling category estimation unit 52 may learn a difference between the tendencies of the ways of thinking.

Moreover, since it is conceivable that, in the case of using only characteristics of word, it is impossible to secure thorough precision, characteristics which are typical to the expression of the cognition may be noticed. Specifically, learning may be carried out with adding a tense, a subject and a voice of verb, and a type of sentence to the original characteristics.

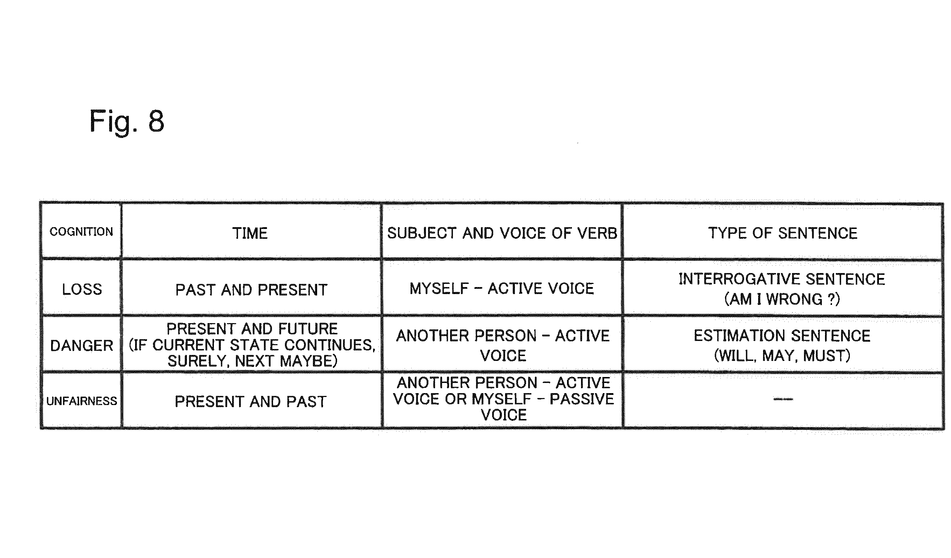

Fig. 8 is an explanation diagram exemplifying characteristics of the automatic thought expression per the category. Fig. 8 shows the tense, the subject and the voice of verb, and the type of sentence of each automatic thought category which are the characteristics of the expression.

For example, the automatic thought expression which is classified into the "loss" category has characteristics shown in the following. That is, the automatic thought expression is often expressed in a form of the past tense and the present tense. Moreover, the automatic thought expression is often expressed in a form that the subject is me, and in a form of the active voice. Moreover, the automatic thought expression is often expressed in a form of an interrogative sentence.

For example, the automatic thought expression which is classified into the "danger" category has characteristics shown in the following. That is, the automatic thought expression is often expressed in a form of the present tense and a future tense. Moreover, the automatic thought expression is often expressed in a form that the subject is another person, and in a form of the active voice. Moreover, the automatic thought expression is often expressed in a form of presumption.

For example, the automatic thought expression which is classified into the "unfairness" category has characteristics shown in the following. That is, the automatic thought expression is often expressed in a form of the present tense and the past tense. Moreover, the automatic thought expression is often expressed in a form that the subject is me, and in a form of the active voice. Or, the automatic thought expression is often expressed in a form that the subject is me, and in a form of the passive voice.

Next, an example of a method for constructing the model will be shown. In the example, it is assumed that a pair of "automatic thought expression and feeling category" is given. The tense, the subject and the voice of verb, and the type of sentence may be given simultaneously. Firstly, the feeling category estimation unit 52 extracts a characteristic word from the automatic thought expression, and calculates frequency of the characteristic word. As a result, it is possible to express the automatic thought as a frequency spectrum of word.

Furthermore, identification precision may be improved by adding an idea shown in the following. For example, the characteristic word may be limited to a word of a specific part of speech such as a noun, an adjective or the like. Moreover, a word which is un-useful for identification such as word "thing"(that is, "mono" or "koto" in Japanese may be registered as a stop word, and then the word may be removed from the characteristic word. Moreover, for example, the characteristic words which are continuous may be regarded as one characteristic word.

As mentioned above, the feeling category estimation unit 52 expresses the automatic thought as a frequency vector of word (hereinafter, referred to as "characteristic word vector") regarding the given pair. Next, the feeling category estimation unit 52 receives the tense, the subject and the voice of verb, and the type of sentence, which are inputted if used, and the characteristic word vector, and learns a relation between these inputs and the feeling category. At this time, the feeling category estimation unit 52 may set the tense, the subject and the voice of verb, and the type of sentence as components of the vector. In this way, the statistical model is constructed.

Next, an example of using the model will be described. Firstly, the feeling category estimation unit 52 extracts a characteristic word from an unknown automatic thought expression. This process may be the same as the process which is carried out when constructing the model. Moreover, in the case that the tense, the subject and the voice of verb, the type of sentence and the like are used at a time of learning for constructing the model, the feeling category estimation unit 52 determines the tense, the subject and the voice of verb, the type of sentence and the like from the unknown automatic thought expression also at the usage time.

For example, in Japanese, the tense and the type of sentence can be identified on the basis of an expression of sentence end for the morphological analysis result. Moreover, the voice of verb can be identified on the basis of existence or non-existence of an expression (for example, existence or non-existence of an expression such as "reru" or "rareru" in Japanese ). Moreover, words which express the first person may be stored in a dictionary or the like in advance, and the feeling category estimation unit 52 may determine the subject by determining whether a word just before the postpositional particle "ga" or "wa" in Japanese is the word of the first-person or not. Here, since the word of the first-person is apt to be omitted in Japanese, in the case that the automatic thought does not include the postpositional particle "ga" or "wa" in Japanese, the feeling category estimation unit 52 may regard the subject as the first-person.

By inputting the tense, the subject and the voice of verb, and the type of sentence, which are inputted if used, and the characteristic vector which is acquired as mentioned above, into a probability model which is constructed in advance, the feeling category estimation unit 52 can estimate statistically to which feeling category a feeling, which is corresponding to the unknown automatic thought expression, is corresponding.

Here, in the case that the feeling category is given as a probability value, the contradiction determination unit 3 in the exemplary embodiment may determine by a distance between two vectors whether being coincident or not coincident.

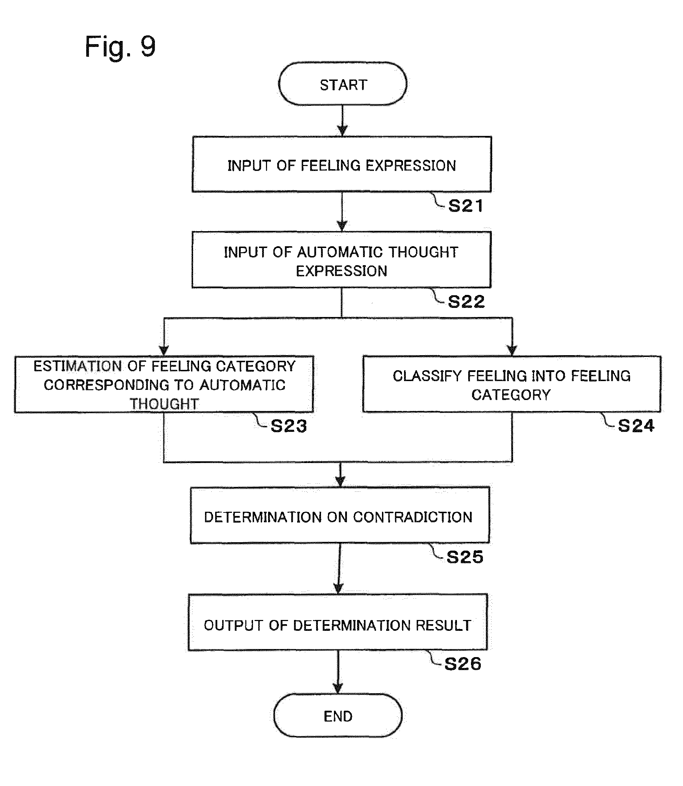

Fig. 9 is a flowchart exemplifying an operation of the mental health care device of the exemplary embodiment. According to the example shown in Fig. 9, firstly, the automatic thought and feeling input unit 51 receives an input of a feeling expression of the counselee regarding an event which beats a heart of the counselee (Step S21). Next, the automatic thought and feeling input unit 51 receives an input of an automatic thought expression of the counselee regarding the event which beats the heart of the counselee (Step S22).

When the automatic thought expression of the counselee is inputted, the feeling category estimation unit 52 estimates a feeling category, which is corresponding to the automatic thought of the counselee, on the basis of the inputted automatic thought expression (Step S23).

Moreover, when the feeling expression of the counselee is inputted, the feeling classification unit 53 classifies the feeling of the counselee into one of the feeling categories.

When acquiring the feeling category which is estimated from the automatic thought of the counselee, and the feeling category which is the result of classifying the feeling of the counselee, the contradiction determination unit 3 determines a contradiction between the automatic thought and the feeling of the counselee (Step S25). Then, the determination result output unit 4 outputs the determination result issued by the contradiction determination unit 3 (Step S26). Here, Step 25 and Step S26 are the same as the Step S13 and Step S14 shown in Fig. 3 according to the first exemplary embodiment respectively.

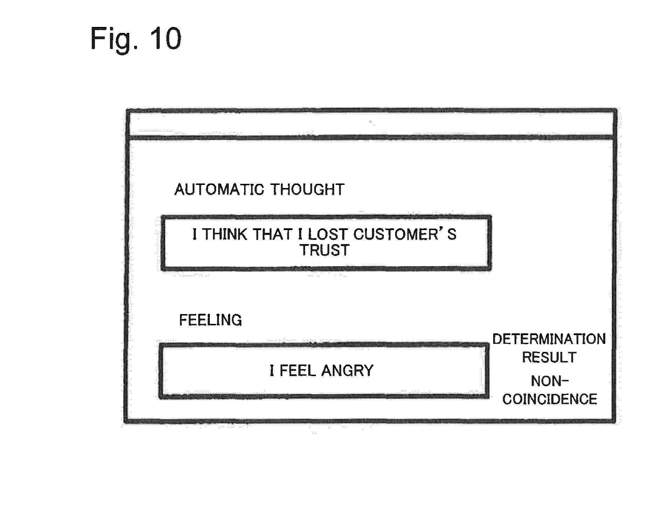

Fig. 10 is an explanation diagram exemplifying the output which indicates the determination result issued by the determination result output unit 4. As shown in Fig. 10, the determination result output unit 4 may output coincidence or non-coincidence, which is indicated in the determination result, together with the input result of the automatic thought and the input result of the feeling.

As mentioned above, since it is determined with no relation to the input form whether there is a contradiction between the automatic thoughts and the feelings or not, and the determination result is outputted according to the exemplary embodiment, it is possible to make the counselor and the counselee perceive whether there is a contradiction between the automatic thoughts and the feelings or not, and consequently it is possible to support accurate counseling.

### Exemplary embodiment 3

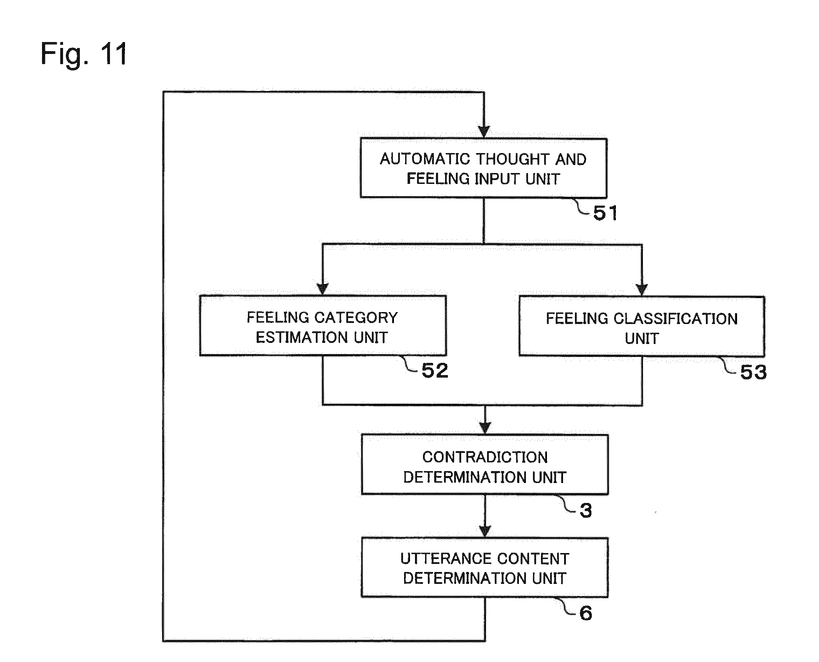

Next, a third exemplary embodiment of the present invention will be described. Fig. 11 is a block diagram exemplifying a configuration of a mental health care support device of the third exemplary embodiment. The configuration of the mental health care support device shown in Fig. 11 is different from the configuration of the second exemplary embodiment shown in Fig. 4 in a point that the determination result output unit 4 is replaced by an utterance content determination unit 6.

The utterance content determination unit 6 determines utterance contents on the basis of the determination result issued by the contradiction determination unit 3.

For example, in the case that a contradiction is found, by utterance contents that "Don't you feel unreasonable in that your thought of 'OO' causes your feeling of 'XX' ?" or "In many cases, a thought of 'OO' may cause to feel 'sad'. Why do you feel 'angry'?", the utterance content determination unit 6 may make the contradiction perceived, and may make the automatic thought and the feeling rechecked. Moreover, the utterance content determination unit 6 may output a message for furthermore investigation.

Moreover, in the case that a contradiction is not found, the utterance content determination unit 6 may output a check message that "We understand that the thought of 'OO' causes you the feeling of 'XX'. Is it correct?".

It is conceivable that determination of the utterance contents has some variations other than the above. Firstly, a case that the automatic thought and the feeling are contradictory to each other will be explained. According to a first variation, the utterance content determination unit 6 outputs a message for inquiring again either of the automatic thought and the feeling. In this case, the utterance content determination unit 6 has a function to determine which of the automatic thought and the feeling should be inquired again. For example, if a situation (an event which beats a heart) is inputted, the utterance content determination unit 6 estimates a feeling category, which is corresponding to the situation, by use of a means to estimates a feeling category, which is corresponding to a situation, from the information. The utterance content determination unit 6 may inquire again a feeling category, which is not coincident to the feeling category corresponding to the situation, out of the feeling category which is corresponding to the inputted automatic thought, and the feeling category of the inputted feeling.

Here, the utterance content determination 6 may use a method, which is similar to the method of the feeling category estimation unit 52, as a method for estimating the feeling category corresponding to the situation. That is, the utterance content determination 6 may construct a statistical model from a preset correct solution pair of one which expresses a situation (hereinafter, referred to as "situation expression"), and a feeling category, and may estimate the feeling category from the optional situation expression by use of the constructed statistical model. Here, the function to estimate the feeling category corresponding to the situation may be installed in the feeling category estimation unit 52, and the feeling category estimation unit 52 may estimate the feeling category corresponding to the situation.

For example, in the case that the feeling category which is corresponding to the situation, and the feeling category of the inputted feeling are not coincident to each other, the utterance content determination unit 6 may output a message for inquiring the feeling again like "Is it correct that this thought makes you feel 'sad' ? Please let us know again your feeling which you had when you got this thought."

Furthermore, for example, in the case that the feeling category which is corresponding to the situation, and the feeling category of the inputted feeling are not coincident to each other, the utterance content determination unit 6 may output a message for inquiring the automatic thought again like "Is it correct that the thought of 'XX' makes you feel 'sad' ? Please let us know again your thought which you got when you felt 'sad'."

According to a second variation, the utterance content determination unit 6 outputs an inquiry message for investigating the automatic thought furthermore. For example, in the case that an automatic thought expression, which is inputted to the feeling category estimation unit 52, include a small amount of information on the automatic thought, there is a case that it is ambiguous which feeling category the automatic thought belongs to. The utterance content determination unit 6 may inquire more specific automatic thought in the case that a difference between the maximum value and the minimum value of the probability in the estimation result issued by the feeling category estimation unit 52 is not larger than a prescribed value, etc. For example, the utterance content determination unit 6 may output a message that "Would you write your thought, which you got when you felt sad, a little bit in detail?".

In the exemplary embodiment, also the automatic thought and feeling input unit 51 may extract the automatic thought and the feeling of the counselee in turn by carrying out a hearing in the dialogue mode.

For example, as a dialogue step 1, the automatic thought and feeling input unit 51 may output a message for extracting "situation" like "What has happened ?", and may receive a text, which is a reply to the message, as the situation expression. Next, as a dialogue step 2, the automatic thought and feeling input unit 51 may output a message for extracting "feeling" like "What kind of feeling did you have at that time ?", and may receive a text, which is a reply to the message, as the feeling expression. Next, as a dialogue step 3, the automatic thought and feeling input unit 51 may output a message for extracting "automatic thought" like "What kind of thought did you get at that time ?", and may receive a text, which is a reply to the message, as the automatic thought expression.

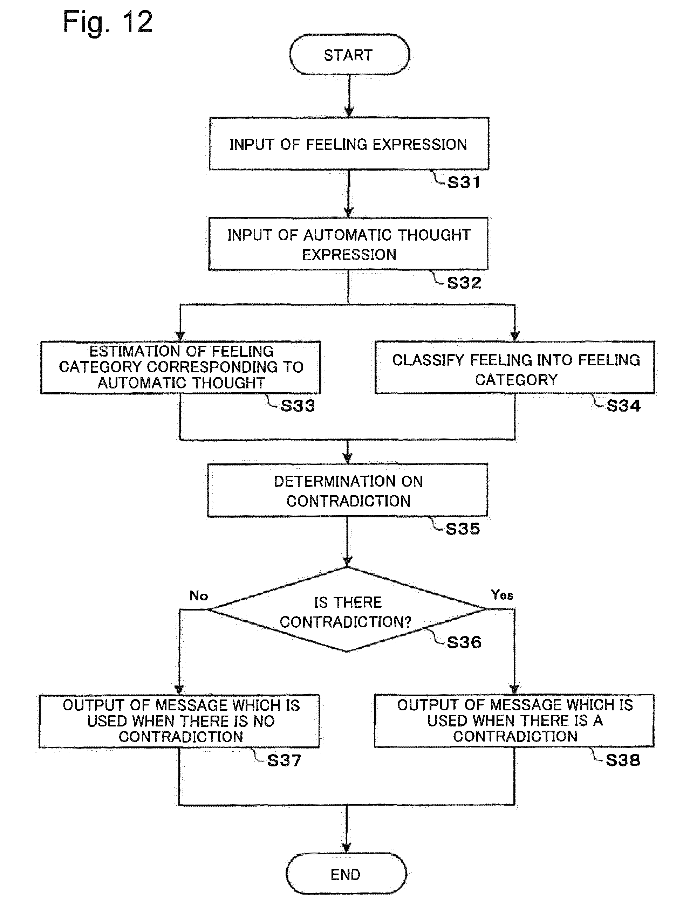

Fig. 12 is a flowchart exemplifying an operation of the mental health care support device of the exemplary embodiment. Operations of Step S31 to S35 are the same as the operations of Step S21 to Step 25 shown in Fig. 9 according to the second exemplary embodiment respectively.

According to the exemplary embodiment, when acquiring the determination result issued by the contradiction determination unit 3, the utterance content determination unit 6 outputs a message based on the determination result (Step S36 to S38). According to the example, the utterance content determination unit 6 checks the determination result (Step S36). In the case that the determination result indicates that there is no contradiction, that is, in the case that the feeling category which is corresponding to the automatic thought, and the feeling category which is corresponding to the inputted feeling are coincident to each other, the utterance content determination unit 6 outputs a predetermined message which is used when there is no contradiction (Step S37). On the other hand, in the case that the determination result indicates that there is a contradiction, that is, in the case that the feeling category which is corresponding to the automatic thought, and the feeling category which is corresponding to the inputted feeling are not coincident to each other, the utterance content determination unit 6 outputs a predetermined message which is used when there is a contradiction (Step S38). Here, the utterance content determination unit 6 may be a means which not only determines the utterance contents based on the determination whether there is a contradiction or not, but also determines utterance contents of a whole of counseling.

As mentioned above, according to the exemplary embodiment, it is possible to acquire an effect, which is similar to the effect of the second exemplary embodiment, without intervention of the counselor. Moreover, since it is possible to carry out recheck and furthermore investigation of the automatic thought and the feeling automatically, it is possible to carry out the effective counseling with reducing man-hours.

Hereinafter, several application examples of the present invention will be explained. The present invention may be arranged, for example, in a system, which carries out the automatic counseling in the dialogue mode on the basis of the cognition reconstruction method, as the means for checking whether there is a contradiction between the automatic thoughts and the feelings in order that an effect of counseling may be improved.

For example, a usual flow of the cognitive behavior therapy is corresponding to a series of steps which are to question the situation, the feeling, the automatic thought, the basis and the counterevidence in turn, and finally to provide adaptive thoughts and to check the current feeling. Then, in a process of carrying out a question sequence based on the cognitive behavior therapy, the present invention can be used for determining whether there is a contradiction between the thoughts and the feelings at the time when the thoughts and the feelings are inputted.

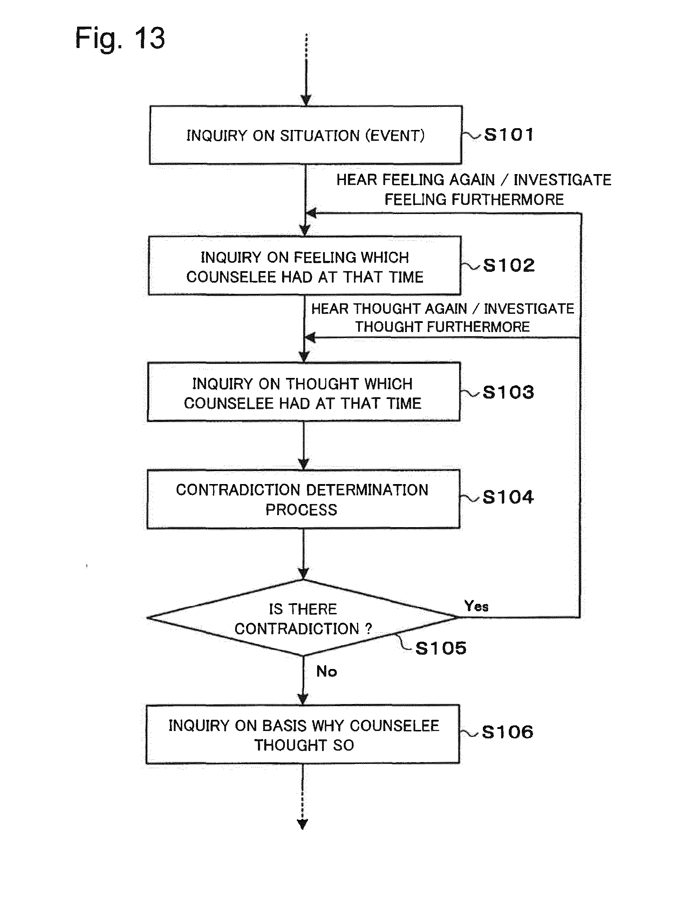

Fig. 13 is a flowchart exemplifying an operation which is carried out in the case that the present invention is applied to a system which carries out self-counseling. As shown in Fig. 13, in a process of extracting the situation, the feeling, the automatic thought, the basis and the counterevidence in turn by a hearing, a process of extracting the situation, the feeling and the automatic thought by the hearing is firstly carried out in turn (Step S101 to S103). Afterward, the contradiction determination process according to the present invention may be carried out (Step S104) without proceeding directly to Step 106, and may change the inquiry sequence, which follows Step S104, according to the result (Step S105).

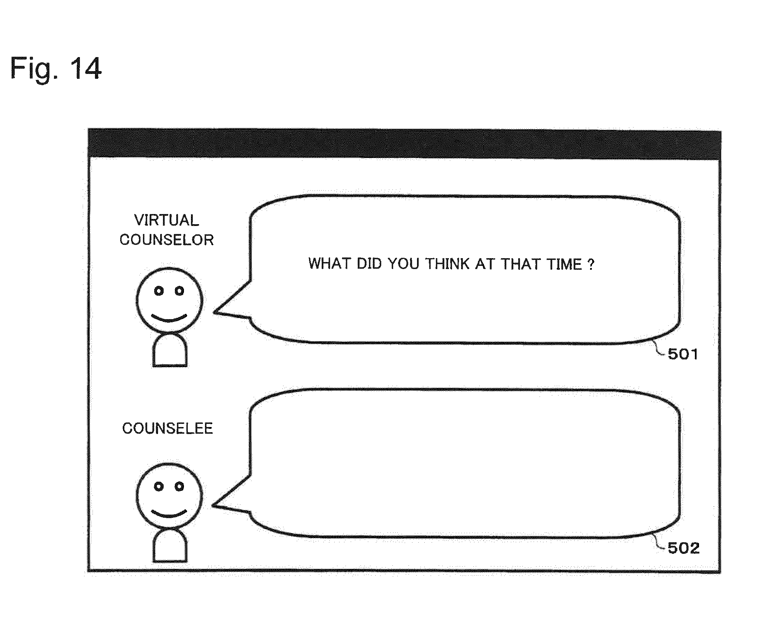

Moreover, each of Fig. 14 to Fig. 17 is an image diagram of a screen exemplifying a content of service according to the present invention.

Fig. 14 is an image diagram of a screen which is displayed in the case that the present invention is applied to an automatic counseling system or a self-care system which the counselee uses. Fig. 14 shows a screen which includes a virtual counselor's message display column 501 and a user's message input column 502. For example, the automatic counseling system prepares a screen such as the above-mentioned screen, and displays a series of messages, which is based on a predetermined scenario, in turn in the message display column 501, and receives the thought and the feeling which are written in the message input column 502 in turn by the user. Afterward, the automatic counseling system carries out the contradiction determination process according to the present invention on the basis of the information, and changes message, which will be displayed in the message display column 501, according to the determination result. By carrying out the above, it is possible to support automatization of the counseling.

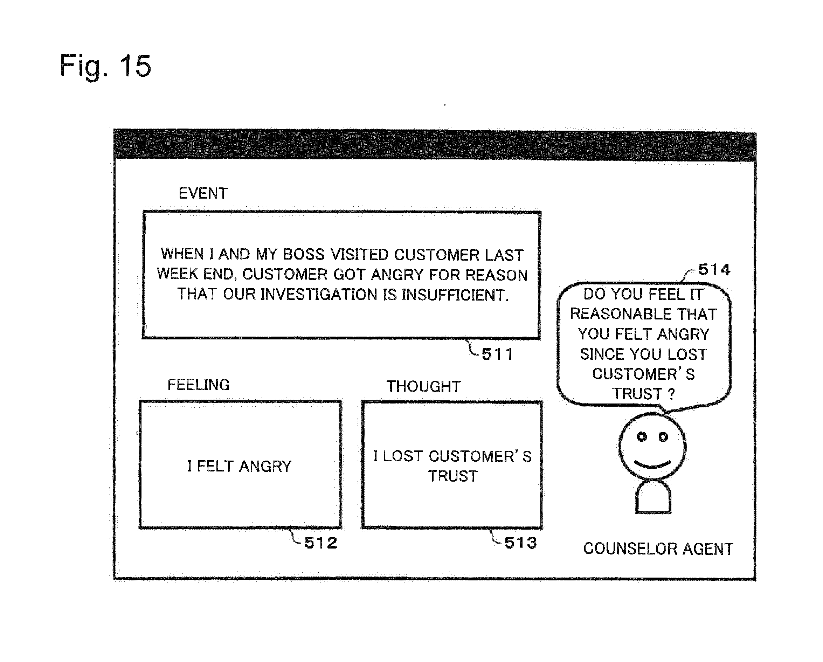

Moreover, Fig. 15 is an image diagram of a screen which is displayed in the case that the present invention is applied to the automatic counseling system or the self-care system which the counselee uses. Fig. 15 shows a screen which includes a user's message input column 511, a user's message input column 512 and a user's message input column 513 which are related to the event, the feeling and the thought respectively, and a counselor-agent's message display column 514. The automatic counseling system prepares a screen such as the above-mentioned screen, and receives the thought and the feeling which are inputted in the message input column 511, the message input column 512 and the message input column 513. Afterward, the automatic counseling system carries out the contradiction determination process according to the present invention on the basis of the information. In the case that a contradiction is found, by outputting the import in the message display column 514 in order to press the user for one more input, the automatic counseling system carries out the input support. By carrying out the above, it is possible to support automatization of the counseling.

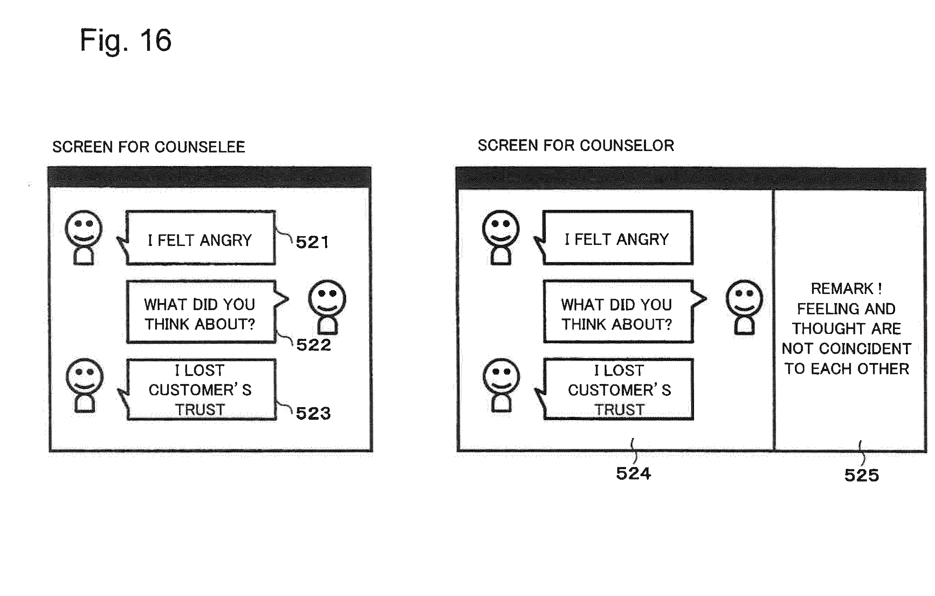

Moreover, Fig. 16 is an image diagram of a screen which is displayed when the counselor diagnoses the counselee in the case that the present invention is applied to a counselor support system. Fig. 16 shows an example that a screen for the counselee and a screen for the counselor are prepared separately. The screen for the counselee includes message input/output columns 521 to 523 in which messages communicating between the counselee and the counselor mode are written in the dialogue. The screen for the counselor includes a message display column 525, in which, in the case that a contradiction is found, the import or the determination result on contradiction is written, other than an area 524 which displays the message input/output columns 521 to 523 of the screen for the counselee. The counselor support system prepares a screen such as the above-mentioned screen, and the user and the counselor input the inquiry message and the reply message in turn in the message input/output columns 521 to 523. The counselor support system carries out the input detection process and the contradiction determination process at a period of time of the input process, and displays an import if a contradiction is found, or the determination result in the message display column 525 of the screen which the counselor uses. By carrying out the above, the counseling by the counselor is supported. As mentioned above, it is possible to make the counselor perceive the contradiction between the thoughts and the feelings of the counselee. As a result, it is possible to improve the effect of counseling. Here, the system may be a remote counseling system in which the counseling is carried out to the counselee by the counselor through a network.

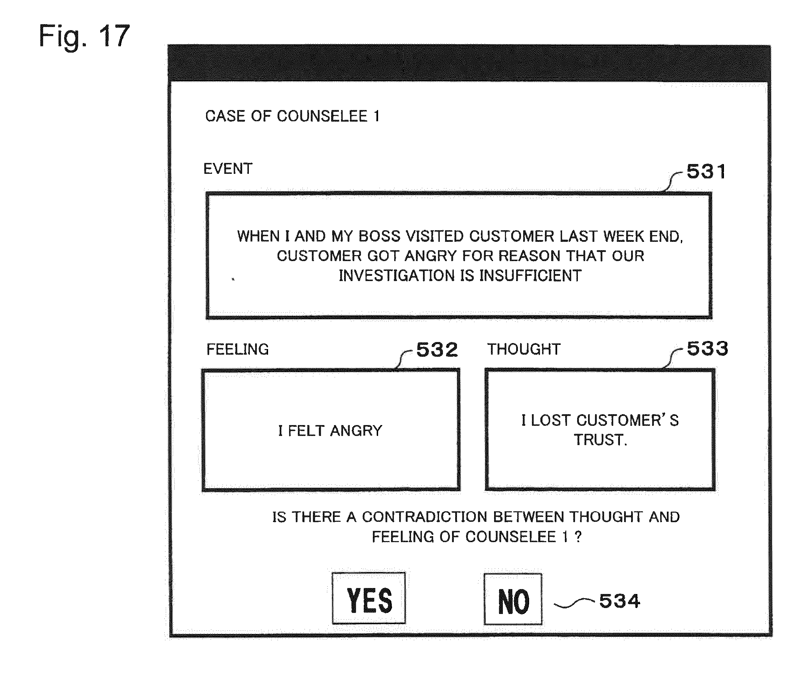

Moreover, Fig. 17 is an image diagram of a screen which is displayed in the case that the present invention is applied to a counselor training system. Fig. 17 shows a screen including message display columns 531 to 533 and a selection button 534. The message display columns 531 to 533 display an event occurring to a counselee, a feeling which the counselee had at that time, and a thought which the counselee had at that time respectively, and the selection button 534 is used for inputting whether there is a contradiction between the thoughts and the feelings of the counselee. The training system prepares a screen such as the above-mentioned screen, and inquires whether an item which is displayed to the counselor on the screen has a contradiction between the thoughts and the feelings of the counselee. The training system receives the selection result which is inputted by handling the selection button 534 in reply to the inquiry, and then carries out the contradiction determination process to the item. The training system may determine whether the inputted selection result is correct or incorrect on the assumption that the result of the contradiction determination process is correct, and may output the determination result. By carrying out a practice of counseling as mentioned above, it is possible to improve an art of counseling.

Moreover, while one device carries out a whole of processes including the information input process and the contradiction determination process until the determination result output process in the above-mentioned exemplary embodiment, it is possible to allot a part of these processes to each device. Hereinafter, several system configuration examples of the present invention will be shown.

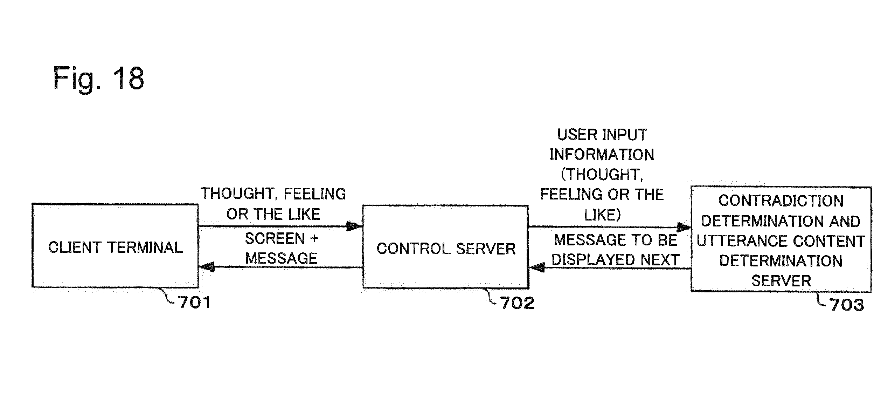

Fig. 18 is a block diagram exemplifying a configuration of a mental health care support system of the present invention. The mental health care support system shown in Fig. 18, which is a system supporting the mental health care by use of a Web system, includes a client terminal 701, a control server 702, and a contradiction determination and utterance content determination server 703.

The client terminal 701, which is a terminal used by the counselee or the counselor, may be a usual terminal which can use a Web system. The client terminal 701 may be, for example, a personal computer which is equipped with an input device such as the mouse, the key-board or the like, and an output device such as the display or the like.

The control server 702 is a Web server which sends and receives various data with the client terminal 701. The control server 702 carries out user authentication, determination on a process related to the user (for example, check of data format inputted by user, or the like), generation of a next screen and the like. Moreover, the control server 702 is connected with the contradiction determination and utterance content determination server 703, which will be mentioned later, so as to be able to exchange data with the contradiction determination and utterance content determination server 703. The control server 702 transfers information (specifically, text which expresses thought, feeling and the like) which is inputted by the user to the contradiction determination and utterance content determination server 703, and then receives message information (determination result on contradiction, next inquiry message or the like), which should be outputted next, from the contradiction determination and utterance content determination server 703.

The contradiction determination and utterance content determination server 703 is a server which carries out the contradiction determination process or the like according to the present invention actually. Moreover, the contradiction determination and utterance content determination server 703 carries out the process of determining the utterance contents according to the determination result on contradiction.

In the case of the configuration shown in Fig. 18, the contradiction determination and utterance content determination server 703 may be equipped with each process unit of the mental health care support device which are shown in each of the exemplary embodiments. Here, in the case that the contradiction determination and utterance content determination server 703 is connected with the client terminal 701 and the control server 702 through a network, the contradiction determination and utterance content determination server 703 may receive desired information from the client terminal 701 and the control server 702 through the network.

Moreover, the control server 701 and the contradiction determination and utterance content determination server 703 may be united to be one device. In this case, the control server 702 may call an execution file of a program which includes the function of each process unit of the mental health care support device shown in each of the exemplary embodiments, and the contradiction determination and utterance content determination server 703 may include the program. Moreover, the contradiction determination and utterance content determination server 703 may operate as individual services. At this time, the control server 702 may provide input the thoughts and the feelings which are received from the client terminal 701 with the contradiction determination and utterance content determination server 703, and may receive the determination indicating whether there is a contradiction or not, and the message to be displayed next, and may outputs them to the client terminal 701. In this case, each function of the contradiction determination and utterance content determination server 703 may be provided as WebAPI.

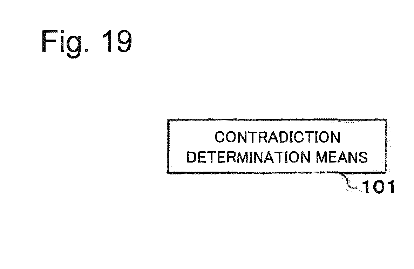

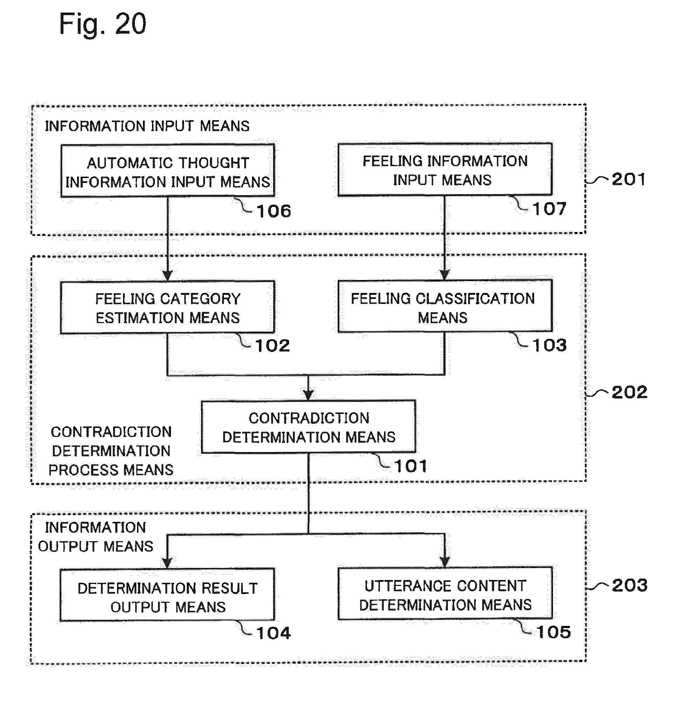

Next, an outline of the present invention will be explained. Each of Fig. 19 and Fig. 20 is a block diagram showing the outline of the present invention. As shown in Fig. 19, a mental health care support device according to the present invention includes a contradiction determination means 101.

The contradiction determination means 101 (for example, contradiction determination unit 3) determines whether there is a contradiction between automatic thoughts and feelings, on the basis of automatic thoughts categories in which either the automatic thoughts of a counselee or automatic thought expressions expressing the automatic thoughts of the counselee are classified into prescribed categories, and feelings categories in which either the feelings of the counselee or feeling expressions expressing the feelings, which the counselee has when an automatic thought occurs, are classified into prescribed categories.

Even in the case that the counselee cannot express the own automatic thoughts and the own feelings correctly with words, or even if there is a contradiction between the thoughts and the feelings of the counselee, since it is determined for the counselor and the counselee whether there is a contradiction between the thoughts and the feeling or not according to the configuration, it is possible to support the counseling accurately by using the determination result.

The contradiction determination means 101 may determine whether there is a contradiction between the thoughts and the feeling of the counselee by determining whether the feeling category corresponding to the automatic thought which is estimated from the automatic thought expressions or the automatic thought categories, and the feeling category into which the feeling of the counselee is classified are coincident to each other or not.

Moreover, as shown in Fig. 20, the mental health care support device of the present invention may include furthermore a feeling category estimation means 102 (for example, feeling category estimation unit 52) for estimating the feeling category, which is corresponding to the automatic thought, from the automatic thought expressions expressing the automatic thoughts of the counselee.

Moreover, as shown in Fig. 20, the mental health care support device of the present invention may include furthermore a feeling classification means 103 (for example, feeling classification unit 53) which, on the basis of the feeling expressions expressing the feelings which the counselee has when an automatic thought occurs, classifies the feelings into prescribed categories.

Moreover, as shown in Fig. 20, the mental health care support device of the present invention may include furthermore a determination result output means 104 (for example, determination result output unit 4) which outputs the determination result issued by the contradiction determination means 101.

Moreover, as shown in Fig. 20, the mental health care support device of the present invention may include furthermore an utterance content determination means 105 (for example, utterance content determination unit 6) which determines a next utterance content on the basis of the determination result issued by the contradiction determination means 101.

Moreover, in the case that the determination result issued by the contradiction determination means 101 indicates that there is a contradiction, the utterance content determination means 105 may recheck the automatic thought or the feeling or may output an inquiry message for investigating the automatic thought and the feeling furthermore.

Moreover, as shown in Fig. 20, the mental health care support device of the present invention may include furthermore an information input means 201 (for example, automatic thought category input unit 1, feeling category input unit 2 or automatic thought and feeling input unit 51) for inputting the automatic thought categories in which either the automatic thoughts of the counselee or the automatic thought expressions expressing the automatic thoughts of the counselee are classified into prescribed categories, and the feelings categories in which either the feelings of the counselee or the feeling expressions expressing the feelings, which the counselee has when an automatic thought occurs, are classified into prescribed categories.

Moreover, the information input means 201 may include an automatic thought information input means 106 for inputting the automatic thought categories in which the automatic thought expressions expressing the automatic thoughts of the counselee, or the automatic thoughts of the counselee are classified into prescribed categories, and a feeling information input means 107 for inputting the feeling categories in which either the feelings of the counselee or the feeling expressions expressing the feelings, which the counselee has when an automatic thought occurs, are classified into prescribed categories.

Moreover, the contradiction determination means 101 may be installed as one process means of a contradiction determination process means 202 which includes the feeling category estimation means 102 and the feeling classification means 103. In this case, by cooperation of each process means included in the contradiction determination process means 202, each process means determines whether there is a contradiction between the automatic thoughts and the feelings on the basis of the automatic thought expressions or the automatic thought categories, and the feeling expressions or the feeling categories which are inputted. Here, the feeling category estimation means 102 and the feeling classification means 103 can be omitted dependently on the inputted information.

Moreover, either of, or both of the determination result output means 104 and the utterance content determination means 105 as an information output means 203 may be installed.

Moreover, the mental health care support device may have a configuration that, when inputting the automatic thoughts categories in which either the automatic thoughts of the counselee or the automatic thought expressions expressing the automatic thoughts of the counselee are classified into prescribed categories, and the feelings categories in which either the feelings of the counselee or the feeling expressions expressing the feelings, which the counselee has when an automatic thought occurs, are classified into prescribed categories, it is outputted whether there is a contradiction between the automatic thoughts and the feelings.

Moreover, the mental health care support device according to the present invention may have a configuration that, when inputting the automatic thoughts categories in which either the automatic thoughts of the counselee or the automatic thought expressions expressing the automatic thoughts of the counselee are classified into prescribed categories, and the feelings categories in which either the feelings of the counselee or the feeling expressions expressing the feelings, which the counselee has when an automatic thought occurs, are classified into prescribed categories, the automatic thoughts and the feelings may be rechecked, or the inquiry message for investigating the automatic thoughts and the feelings furthermore may be outputted.

While the invention according to the present application has been explained with reference to the exemplary embodiment and the example, the invention according to the present application is not limited to the exemplary embodiment and the example. Various changes, which a person skilled in the art can understand, can be added to the composition and the details of the invention according to the present application within the scope of the invention according to the present application.

This application is based upon and claims the benefit of priority from Japanese Patent Application No. 2012-209523, filed on September 24, 2012, the disclosure of which is incorporated herein in its entirety by reference.

### Industrial Applicability

The present invention is preferably applicable to not only the mental health care support system for the counselee but also the mental health care support system for the counselor.

### Reference signs List

- 1: automatic thought category input unit
- 2: feeling category input unit
- 3: contradiction determination unit
- 4: determination result output unit
- 51: automatic thought and feeling input unit
- 52: feeling category estimation unit
- 53: feeling classification unit
- 6: utterance content determination unit
- 701: client terminal
- 702: control server
- 703: contradiction determination and utterance content determination server
- 101: contradiction determination means
- 102: feeling category estimation means
- 103: feeling classification means
- 104: determination result output means
- 105: utterance content determination unit
- 106: automatic thought information input means
- 107: feeling information input means
- 201: information input means
- 202: contradiction determination process means
- 203: information output means

## Claims

1. A mental health care support device, **characterized by** comprising:
contradiction determination means for determining whether there is a contradiction between an automatic thought and a feeling, on the basis of an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category.

2. The mental health care support device according to claim 1, wherein:
the contradiction determination means determines whether or not there is the contradiction between the automatic thought and the feeling by determining whether or not the feeling category corresponding to the automatic thought which is estimated from the automatic thought expression or the automatic thought category, and the feeling category into which the feeling of the counselee is classified are coincident to each other.

3. The mental health care support device according to claim 1 or claim 2, further comprising:
feeling category estimation means for estimating the feeling category corresponding to the automatic thought on the basis of the automatic thought expression expressing the automatic thought of the counselee.

4. The mental health care support device according to any one of claim 1 to claim 3, further comprising:
feeling classification means for classifying the feeling into the prescribed category on the basis of the feeling expression expressing the feeling of the counselee when the automatic thought occurs.

5. The mental health care support device according to any one of claim 1 to claim 4, further comprising:
determination result output means for outputting a determination result issued by the contradiction determination means.

6. The mental health care support device according to any one of claim 1 to claim 4, further comprising:
utterance content determination means for determining a next utterance content on the basis of the determination result issued by the contradiction determination means.

7. The mental health care support device according to claim 6, wherein
the utterance content determination means, in the case that the determination result issued by the contradiction determination means indicates that there is the contradiction, rechecks the automatic thought or the feeling, or outputs an inquiry message for investigating the automatic thought or the feeling furthermore.

8. The mental health care support device according to any one of claim 1 to claim 7, further comprising:
information input means for being inputted the automatic thought expression expressing the automatic thought of the counselee or the automatic thought category in which the automatic thought of the counselee is classified into prescribed category, and the feeling expression expressing the feeling of the counselee when the automatic thought occurs or the feeling category in which the feeling of the counselee is classified into prescribed category.

9. A mental health care support device, **characterized by** comprising,
when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, outputting whether there is a contradiction between the automatic thought and the feeling.

10. A mental health care support device, **characterized by** comprising,
when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, rechecking the automatic thought or the feeling, or outputting an inquiry message for investigating the automatic thought or the feeling furthermore.

11. A mental health care support system, **characterized by** comprising, a server connected with a client terminal, which is operated by a counselee or a counselor, through a network; and
the server includes contradiction determination means for determining, when receiving an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category, whether there is a contradiction between an automatic thought and a feeling, on the basis of the received automatic thought expression or the automatic thought category, and the feeling expression or the feeling category.

12. A mental health care support method, **characterized by** comprising,
when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, determining whether there is a contradiction between the automatic thought and the feeling on the basis of the inputted automatic thought expression or the automatic thought category, and the feeling expression or the feeling category.

13. A mental health care support program, **characterized by** causing a computer to execute:
a process for, when an automatic thought expression expressing the automatic thought of the counselee or an automatic thought category in which the automatic thought of the counselee is classified into prescribed category, and the feeling expression expressing the feeling of the counselee when the automatic thought occurs or a feeling category in which the feeling of the counselee is classified into prescribed category are inputted, determining whether there is a contradiction between the automatic thought and the feeling on the basis of the inputted automatic thought expression or the automatic thought category, and the feeling expression or the feeling category.
